Europäisches Patentamt

European Patent Office

Office européen des brevets

(11) Publication number: **0 095 291**
**B1**

# EUROPEAN PATENT SPECIFICATION

(45) Date of publication of patent specification: **15.03.89**

(21) Application number: **83302731.1**

(22) Date of filing: **13.05.83**

(51) Int. Cl.⁴: **G 01 N 33/00, G 01 N 33/48,**
**G 01 N 33/66, A 61 M 1/36**

(54) Continuous measurement of the concentration of a predetermined ingredient in a diluted liquid.

(30) Priority: **15.05.82 JP 82009/82**

(43) Date of publication of application:
**30.11.83 Bulletin 83/48**

(45) Publication of the grant of the patent:
**15.03.89 Bulletin 89/11**

(84) Designated Contracting States:
**BE DE FR GB IT**

(56) References cited:
**CA-A-1 040 271**
**DE-A-2 720 210**
**DE-A-3 039 126**
**US-A-4 151 845**
**US-A-4 206 755**

(73) Proprietor: **KABUSHIKI KAISHA KYOTO DAIICHI**
**KAGAKU**
**57 Nishiaketa-cho Higashikujo, Minami-ku**
**Kyoto-shi Kyoto 601 (JP)**

(72) Inventor: **Uchigaki, Takatoshi**
**5, Kamikomahigashi-tsukurimichi**
**Yamashiro-cho Soraku-gun Kyoto (JP)**
Inventor: **Hyodo, Hiroshi**
**28, Oike-cho, Kisshoin**
**Minami-ku Kyoto-shi Kyoto (JP)**
Inventor: **Iwase, Teijiro**
**3-F-814, Mukojima-New Town 151-30,**
**Ninomaru-cho**
**Mukojima Fushimi-ku Kyoto-shi Kyoto (JP)**

(74) Representative: **Seaborn, George Stephen et al**
**c/o Edward Evans & Co. Chancery House 53-64**
**Chancery Lane**
**London WC2A 1SD (GB)**

Courier Press, Leamington Spa, England.

**Description**

Field of the invention

The present invention relates to monitoring of the concentration of a predetermined or specified ingredient in a first liquid the first liquid being diluted and the concentration of the specified ingredient in the diluted liquid being measured to determine the concentration of the ingredient in the first, undiluted liquid.

Background of the invention

It is common when measuring the concentration of a specified ingredient (a substance to be measured) contained in a liquid to be tested, to supply to a measuring device instead of the single liquid to be tested, a mixture of the liquid to be tested and some other liquid (in other words, to supply the liquid to be tested diluted by some other liquid). The purpose of such mixing (dilution) may be, for example, to effect pH-adjustment, to remove interfering matters, to prevent coagulation in a blood sample or simply to reduce the concentration of the specified ingredient. The dilution of the liquid to be tested is liable to cause errors in the measured values of concentration owing to variations (errors or scattering) of the mixing rate (dilution ratio). In particular, when the liquid to be tested is flowing through a channel and is continuously monitored there occurs often the difficulty of keeping the dilution ratio constant, fluctuations of the dilution ratio being directly expressed in errors of the measured value of concentration.

To give an instance, in an apparatus wherein the liquid is blood is drawn from a vein of the subject (patient) by the use of a double-current catheter while infusing an anticoagulant as diluent in order to continuously and automatically monitor the concentration of a specified ingredient (for example, glucose) contained in the blood, there is generally employed, to feed anticoagulant to the catheter a tube pump that subjects the anticoagulant to pulsations at only a low rate, is of little dead volume and is able to continuously feed anticoagulant at a low rate.

However, such a pump has the defect that the rate of discharge of anticoagulant changes with the passage of time and accordingly the dilution ratio of the blood fluctuations in the course of continuing with the measurement for many hours. Therefore it is hard to obtain an accurate measured value of the concentration of the specified ingredient. This is attributable, for example, to the deterioration or deformation with the passage of time of the pump tube which is continuously being squeezed or to the change in inner diameter of the tube due to the adhesion of the constituents of the liquid.

With a view to avoiding such a problem, there has heretofore adopted a method in which the double-current catheter is detached from the patient during the course of the measurement, is made to suck in a reference liquid, and, after dilution has been recalculated, the catheter is reattached to the patient. When using this method, however, the measurement is not only discontinued for a while but also accompanied by troublesome operations. Besides due consideration must be paid to the possibility of infection or injury to the subject (patient) which arise from such operations. In order to eliminate such weak points, there is disclosed in the Japanese Patent Application Disclosure No. 135795 of 1977 a technique in which variations in the dilution ratio are compensated for by comparing blood separately drawn from a vein of the patient by the use of a syringe with the blood sample drawn from the catheter. However, the separate drawing of blood by syringe not only causes pain to the patient but, what is worse, increases the quantity of blood loss. Furthermore the blood sample drawn by syringe has to be treated anticoagulantly within a prescribed short time and has to be diluted accurately to a prescribed dilution ratio. Thus the treatment of the blood sample is necessarily very intricate.

DE—A—3 039 126 discloses apparatus comprising the features of the pre-characterising part of the claim given below.

As mentioned above, the prevalent known methods of compensating for variations in the dilution ratio during continuous drawing of blood from a patient have the drawbacks that pain and danger of infection to the patient are caused, much labour is required and the methods are very complicated, thereby giving rise to the possibility of errors attributable to operating mistakes occurring.

Summary of the invention

It is an object of this invention to enable such defects as mentioned above to be eliminated and to provide apparatus for monitoring the concentration of a specified ingredient of a first liquid, the concentration of the ingredient in a second liquid formed by diluting the first liquid being measured to determine the concentration of the specified ingredient in the first, undiluted liquid, the concentration of the specified ingredient in the first liquid being stably and accurately determined for many hours at the same time the first liquid is being diluted.

Another object of this invention is to provide apparatus by means of which compensation for variations in the dilution ratio can be done automatically at periods prescribed in advance, and measurement of high accuracy can be performed continuously, and, in addition, to provide also apparatus in which compensation for variations in the dilution ratio by the reference liquid to be conducted before the commencement of the measurement is dispensable, or compensation for variations in the dilution ratio can be made even by using a reference liquid having an unknown concentration of the specified ingredient.

A further object of this invention is to provide apparaus which has the ability to compensate for variations in the dilution ratio while it is still receiving the first liquid from a double-current catheter on a human body (patient) and without the necessity of any human-intervention.

The invention resides in the principle of finding a real dilution ratio while changing the rate of supply of liquid being supplied to either of or both of two tube pumps which respectively pump either the first liquid and a diluent or a diluent and a mixture of the first liquid and the diluent.

In accordance with the present invention there is provided apparatus for monitoring the concentration of a predetermined ingredient in a first liquid, the apparatus comprising:

a first tube pump for supplying diluent to be mixed with the first liquid to give a mixed liquid;

a second tube pump for supplying the first liquid or the mixed liquid;

first and second means for respectively driving the first and second tube pumps, the speed of at least one of the pumps being variable;

means for measuring the concentration of the ingredient in the mixed liquid and providing an output signal corresponding to the measured concentration;

characterized by the apparatus further comprising:

means for receiving said output signal, for varying the speed of said at least one speed-variable tube pump to change the dilution ratio of the mixed liquid, for determining from the measured concentrations before and resulting from such change of dilution ratio and from the speed change of the tube pump the value of the dilution ratio before or resulting from the change of dilution ratio and determining from such value the concentration of the predetermined ingredient in the first liquid.

Brief description of the drawings

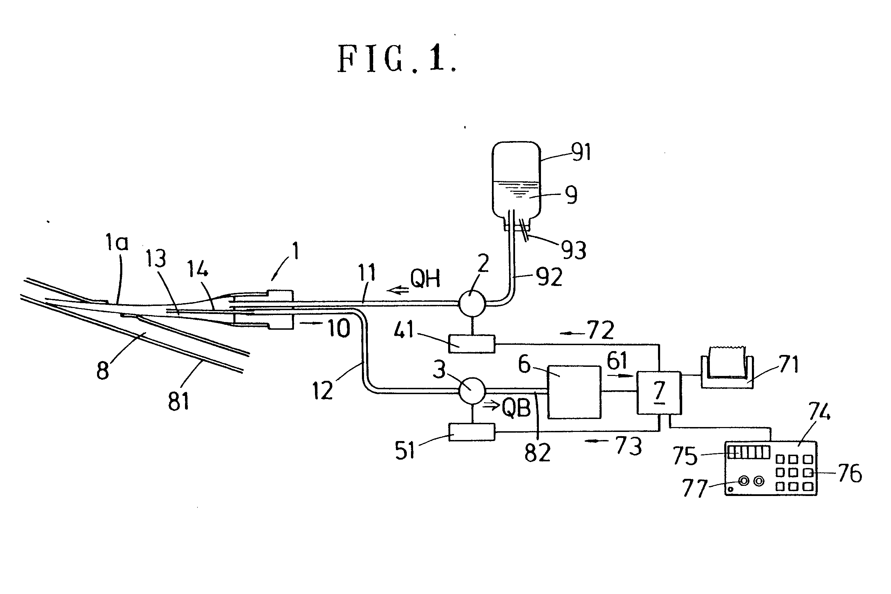

Figure 1 is a schematic drawing showing an apparatus according to the invention;

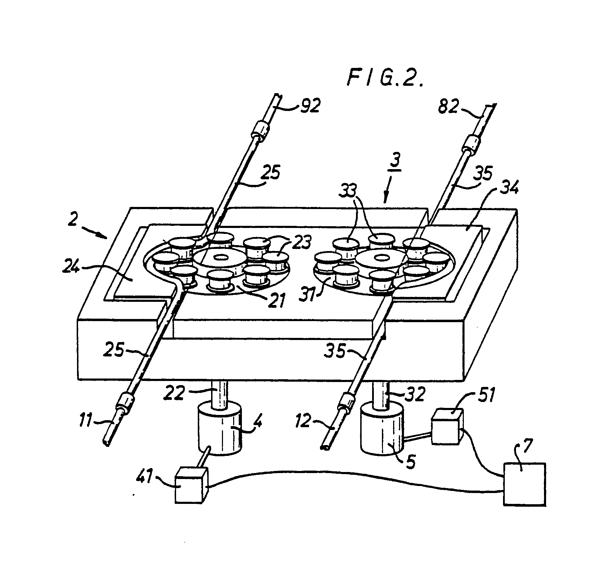

Figure 2 is a perspective view of a part of a tube pump of the apparatus;

Figure 3 is a plan view showing a modification of the blood-drawing section; and

Figure 4(a) and (b) are schematic drawings showing parts of apparatus according to the invention.

Detailed description

The invention is described below with reference to continuous measurement of blood-sugar value (glucose concentration) in blood sampled by using a double-current catheter.

The apparatus comprises a double-current catheter 1, catheter tubes 11 and 12, tube pumps 2 and 3, first and second pulse motors 4 and 5 for driving the tube pumps 2 and 3 respectively, a measuring section 6, a microcomputer 7 and an indicating device 71. Pulse motor-driving circuits 41 and 51 are connected to the first and second motors 4 and 5 respectively. Arrows 72 and 73 denote controlling signals (speed change-command signals) which are emitted from the microcomputer 7. In accordance with these signals 72 and 73 the rate of revolution (speed) of the tube pumps 2 and 3 can be changed. Further the tube pumps 2 and 3 comprise rotors 21 and 31 respectively, rotor shafts 32 and 33, rollers 23 and 33, pump heads 24 and 34, and pump tubes 25 and 35. The double-current catheter 1 is inserted into the inside of a canula 1a whose tip is to be detained in a vein. The space between the catheter 1 and the canula 1a forms an anticoagulant reservoir 13. Anticoagulant 9 is supplied through an anticoagulant-feeding tube 92 and a first catheter tube 11 into the anticoagulant reservoir 13 by the feeding action of the first tube pump 2. An air-introducing needle 93 is provided.

A blood sample (liquid to be tested) 8 is drawn in, at the tip part of a suction tube 14 of the catheter by the sucking action of the second tube pump 3 while being mixed with (and diluted by) the anticoagulant 9, forming a mixed liquid 10. The mixed liquid 10 passes through the suction tube 14 of the catheter, the second catheter tube 12, and a mixed liquid-feeding tube 82, and reaches the measuring section 6. In the measuring section 6, a measuring signal 61 corresponding to the glucose concentration in the mixed liquid 10 is emitted from, for example, an immobilized enzyme film electrode. The measuring signal 61 is put into an arithmetic memory system of the microcomputer 7 from which the blood-sugar value is calculated on the basis of the operation described below. This value is displayed on the indicating device 71.

The dilution ratio δ is the ratio by volume of the mixed liquid 10 to the blood sample 8. Supposing the quantity of discharge of the tube pump 2 is QH and that of the tube pump 3 is QB, the following equation can be obtained.

$$\delta = QB/(QB - QH)$$

Further, the dilution ratio δ also is equal to the ratio of the blood-sugar value X in the blood sample 8 and also to the glucose concentration G in the mixed liquid 10. Therefore:

$$\delta = X/G \qquad (1)$$

(In the following, the blood-sugar value X means the glucose concentration in the blood sample and is to be distinguished from the glucose concentration G in the mixed liquid).

From the above two equations, the following equation, which gives blood-sugar value X, may be derived:

$$X = \delta \cdot G = QB \cdot G/(QB - QH) \qquad (2)$$

3

Conventionally, when the measuring signal 61 corresponding to this G is obtained, the value of δ is regarded as a definite or constant one obtained ahead of time and, on this basis, the blood-sugar value is displayed on the indicating device 71 as X. In such a case, possible fluctuations of the value of δ are not taken into consideration, even though they give rise to errors in the value of X displayed on the device 71. Aside from this, there is adopted a method in which compensation for variations of the dilution ratio is done by using a reference liquid having a known concentration of glucose or by using a blood sample drawn separately by the use of a syringe, as done in the conventional technique mentioned above, as a substitute for the blood sample continuously drawn.

Contrary to this, in the present invention, values of δ given by the equation (1) are successively stored in the microcomputer 7. The values of δ may be stored by various methods, such as by manually inputting or automatically memorizing the values obtained by using a reference liquid having a known glucose concentration is known in place of the blood sample 8 or by the measurement process described below.

After the dilution ratio $\delta_0$ obtained by using the reference liquid whose concentration of glucose is known has been stored in the microcomputer 7 prior to the measurement, the double-current catheter 1 is set in a vein of the patient in order to commence the measurement.

However, the above-mentioned QH and QB fluctuate with passage of time for the aforesaid reasons, in proportion to which the value of δ varies, too. Even if such a variation would be insignificant yet its errors can not be ignored when the measurement continues to be done for long hours.

The method described below in accordance with the invention comprises finding real dilution ratios at any point of time, making the microcomputer 7 correctively memorize the above real dilution ratios in succession, and obtaining continuously accurate blood-sugar values X's, on the basis of the following principle:

First, supposing the blood-sugar value of the blood sample 8 at one point of time $T_1$ is $X_1$, the glucose concentration in the mixed liquid 10 is $G_1$, the rates of discharge of the tube pumps 2 and 3 are $QH_1$ and $QB_1$, respectively, and the real dilution ratio is $\delta_1$, the following equation (3) is derived by the equation (2);

$$X_1 = \delta_1 \cdot G_1 = QB_1 \cdot G_1 / (QB_1 - QH_1) \qquad (3)$$

Then, if the speed (rate of revolution) of the first tube pump 2 is increased forcedly by N times by modifying the controlling signal 72, the quantity of discharge of the tube pump 2 becomes also N times because the number of revolution of the pump and its quantity of discharge stand in proportional relation each to other. Supporting the glucose concentration in the mixed liquid 10 under the above condition is $G_1$, the blood-sugar value of the blood sample 8 is $X_1'$, the quantities of discharge of the tube pumps 2 and 3 are $QH_1'$ and $QB_1'$, respectively, and the real dilution ratio is $\delta_1'$, the following equation will be obtained similarly from the equation (2),

$$X_1' = \delta_1' \cdot G_1' = QB_1' \cdot G_1' / (QB_1' - N \cdot QH_1') \qquad (4)$$

In the case of the interval of the measurement being short, $X_1$ and $X_1'$ are substantially equal in light of the characteristic of the change with the passage of time of the blood-sugar value, and similarly the degrees of deformation of the pump tubes 25 and 35 or the change of inner diameter thereof are negligible, so that $QB_1$ and $QB_1'$ as well as $QH_1$ and $QH_1'$ can be regarded as equal, respectively, wherefrom the real dilution ratio can be determined by eliminating the quantities of flow of the tube pumps from equations (3) and (4).

That is, from these relations and the equations (3) and (4) will be obtained the following equation:

$$\delta_1 = X_1 / G_1 = (NG_1 - G_1') / G_1 (N - 1) \qquad (5)$$

Now, the real dilution ratio $\delta_1$ calculated on the basis of the above equation (5) is stored in the microcomputer 7 in place of the dilution ratio $\delta_0$ mentioned at first. The blood-sugar value X which is calculated from this time on by using this dilution ratio $\delta_1$ by the equation (2) is displayed on the measuring device 71.

Then follows that at certain periods of time $T_2$, $T_3$... the speed (rate of revolution) of the tube pump 2 is forcedly altered similarly to the above to obtain $\delta_2$, $\delta_3$... which are stored in succession. In this connection, the rate of revolution of the tube pump 2 may at each period of time be increased by N times temporarily at each period of time and immediately be restored to the original state (that is, reduced to 1/N), or after the measurement has been done the rate of revolution of the pump 2 may be maintained at N times its original rate until time $T_2$ and then decreased by 1/N times.

Therefore, even though the dilution ratio fluctuates owing to the variation with the passage of time of the rate of discharge of each of the tube pumps 2 and 3 at the time of the measurement, the dilution ratios stored in the microcomputer 7 are successively corrected to a value very close to the real dilution ratio at all times so that it is possible to measure the blood-sugar value accurately and continuously. The dilution ratio may be stored in the microcomputer 7 by various methods, for example, by inputting manually numerical values displayed (coming out) on the indicating window 75 of the operating section 74 by using the input key 76, or by automatically storing them by operating a dilution ratio-compensating switch 77.

A preferred embodiment of the present invention has been described above with reference to the drawings. However, the invention is not limited thereto and various modifications such as described below may be made within the scope of the invention.

Although, in the above-described example, the number of revolutions of the tube pump 2 on the anticoagulant-feeding side is increased forcedly by N times in order to find the respective glucose concentration values ($G_1$, $G_1'$) in the two kinds of mixed liquids, it is equally satisfactory if the number of revolution of the said pump 2 is increased by 1/N time, or the tube pump 3 on the mixed liquid side is changed in speed or both tube pumps 2 and 3 are changed in speed alternatively or simultaneously. It is also satisfactory if this compensation (recheck) of the dilution ratio is manually instructed to the apparatus at any period of time. Alternatively, the microcomputer 7 may be set so as to conduct automatically the compensation of the dilution ratio, for example, after 30 minutes, after 60 minutes and so on.

It is further possible to change the rate of revolution of either of the tube pumps 2 and 3 in several stages, for example, by increasing it by N times at one period of time, or on top of that by M times at period of time. To be short, it does not matter even if the mixing rate (dilution ratio) is made to be changed according as the respective rates of revolution of either of or both of the tube pumps are changed before and after a certain period of time, thereby the respective valves Gs becoming findable.

Further, in the above-mentioned example, although the real dilution ratio was found by the first speed-change operation, it suffices if the real dilution ratio is decided on the average of the values of all dilution ratios obtained by repeating such speed-change operations as above several times at a certain period of time, (in this case, it is permissible to apply the combination of various kinds of speed-change operations mentioned above instead).

In the aforementioned example, the value of the blood sugar in the blood sample is arranged so as to be calculated by using the real dilution ratio obtained at the then period of time under the speed change operation of the tube pump until the next time as it is. This calculation can be also done in such a manner as follows:

On one occasion, the value of the blood-sugar is compensated by using the dilution ratio obtained under the said speed change while tracing back partly before the speed-changing period of time (for example, up to the middle between the present speed-changing and last compensating periods of time); or on another occasion, while changing proportional-distributively the two dilution ratios obtained at different periods of time, the value of the blood-sugar between both periods of time is compensated successively by using the said changing dilution ratios retroactively; and so on. In these cases, however, the compensation is made after finding the blood-sugar value, so that it is necessary to make the value not yet compensated to be once stored. Such a modification is helpful especially in the case where the dilution ratio fluctuates sharply.

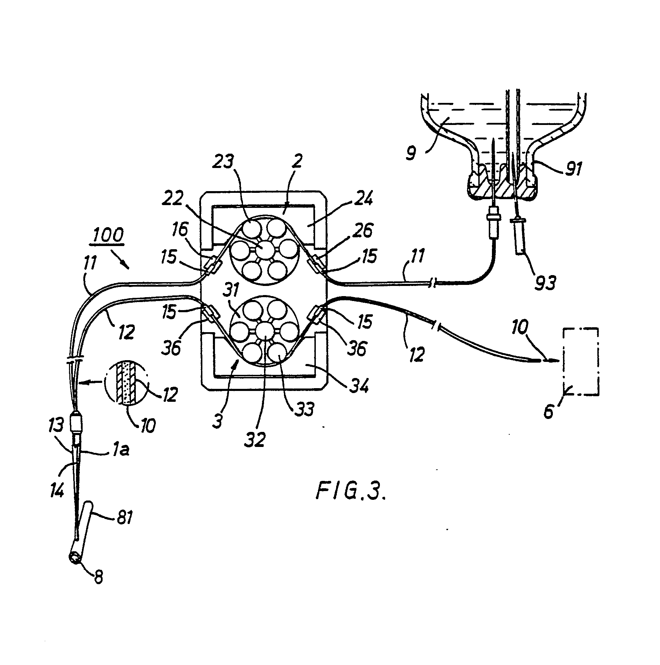

The driving source of the tube pumps 2 and 3 is not limited only to pulse motors, but may be anything that is provided with the function of being able to obtain a desired rate of revolution, and may comprise a speed-change gear. Further, as an example of a modification of the blood-drawing section, there can be used such a double-current catheter 100 which is designed and arranged so as to also perform the duties of both the pump tube 25 and anticoagulant-feeding tube 92 and the tube 35 and mixed liquid-feeding tube 82 in the former example, by making each of the cathether tubes 11 and 12 longer, as shown in Figure 3.

Stoppers 15' are attached to the catheter tube 11 and 12. Stopper receivers 26 and 36 are provided in the tube pumps 2 and 3. The double-current catheter 100 has various advantages that it is subject to less sedimentation of foreign matter because of both its tubes 11 and 12 having no more than the end parts in their connecting points, that it has less apprehension of the tubes being clogged, that the exchange of the catheter is made very easily and promptly, that the damage of the contact places with roller attributable to the speed change of the pumps 2 and 3 offers no problem on exchangeable independently in each catheter, and so on.

In all of the above-mentioned examples, it is arranged in such a manner that the real dilution ratios varying progressively are rechecked while forcedly changing the rate of revolution of the tube pumps at an optional period of time, that the values thus obtained are correctively stored in the microcomputer 7 in due succession, and that a more accurate value of the blood-sugar comes to be found. Comparing this obtained by using the initial reference liquid at the beginning of measuring, the changing portions are made to be fed back to, for example, the pulse motor-driving circuits 41 and 51, whereby it is also possible to compensate the dilution ratio in such a manner as to return to the original dilution ratio. On the other hand, because the real blood-sugar value at the above period of time can be found by the aforementioned equation (5), it does not matter if arranging to calculate only the real blood-sugar values, not the real dilution ratios, at every regular interval and to display them.

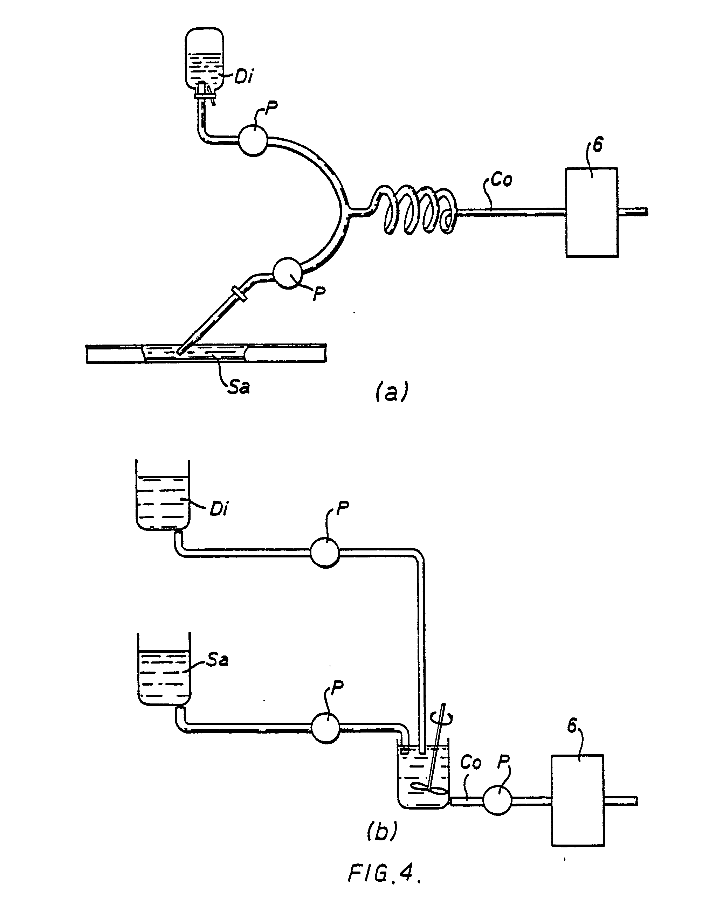

Further, the apparatus according to the present invention may be used in the field of chemical analysis for continuously measuring specified ingredients, including the aforesaid analysis of blood. In addition, this apparatus is not limited to the case where the double-current cathether is used in the manner as mentioned above; it may be also applied to the case where the progression of the mixed liquids ($C_o$) which are supplied to the measuring section 6 is observed with the passage of time: one mixed liquid has been obtained by transferring the liquid to be tested (Sa) and the diluent (Di) by the respective tube pumps (P) while mixing on the way, as shown in Figure 4(a), and another mixed liquid by supplying the liquid to be tested (Sa) and the diluent (Di) by means of the respective pumps (P) into the mixing vessel (C) while stirring thereat.

As described above, the compensating of the dilution ratio by means of the present invention is such a one in which, in the case of finding the concentration of a specified substance contained in the liquid to be tested through the measurement of the concentration of the mixed liquid which is made by mixing the liquid to be tested with the diluent by the use of two tube pumps, the real dilution ratio and therewith the accurate concentration are found easily and certainly by making forcedly the quantity of liquid-feeding of either or both of the tube pumps. Accordingly, even when such a measurement as the continuous measuring of the blood-sugar value is made for long hours, it becomes possible to perform the compensation (recheck) of the dilution ratio in a state of loading the double-current catheter on the patient as it is. As a result, there is no necessity of detaching the catheter on the middle of the measurement and of dipping it in the reference liquid. The use of this catheter does not require drawing separately blood by a syringe for submitting to compensation; it is safe and hygenic because of the nonintervention of manpower operation and does not give extra pain to the patient. All things considered, this catheter provides a technique of very high utility in the clinical field. Further, this invention is able to automate the compensating motion and to perform the automatic compensation of the dilution ratio at the present period, so that it has become possible for the analyser and the technician to make the measurement of high accuracy continuously though staying away remote from the place where the measuring apparatus is set up, thereby a large economy of time and labour can be brought about. What is more, in the apparatus of our invention, it is also possible to eliminate various kinds of adverse influences such as, for example, by the deterioration and deformation of the tube pumps with the passage of time, or the change in inner diameter of the tubes similarly with the passage of time, and consequently the apparatus is suitable for long use, with the result that the total cost of the measuring operation be decreased.

Further, in the invention, it is not necessary that the reference liquid to be used for the compensation of the dilution ratio which will be performed prior to the commencement of the measurement must have a known concentration of the specified ingredient; it suffices if only it is adjusted nearly to a desired concentration. That is, it becomes possible to use a reference liquid of unknown concentration by making the compensation of the dilution ratio according to the present invention. This means that the invention is very convenient in the points that the reference liquid can be used as it is even in the case of being ambiguous as to whether it has such an accurate concentration as prescribed or not, and that there can be used a sterilized reference liquid which is adjustable easily as to its concentration of the specified ingredient by using e.g. physiological saltwater or glucose injection commercially available on the market. If circumstances require, omitting the compensation of the dilution ratio performed by the use of the reference liquid, it is also practicable to make the compensation of the dilution ratio while using the liquid to be tested itself such as the blood of the patient directly after the catheter has been inserted into him.

## Claim

Apparatus for monitoring the concentration of a predetermined ingredient in a first liquid (8), the apparatus comprising:

a first tube pump (2) for supplying diluent (9) to be mixed with the first liquid to give a mixed liquid (10);

a second tube pump (3) for supplying the first liquid or the mixed liquid;

first and second means (4, 5) for respectively driving the first and second tube pumps, the speed of at least one of the pumps being variable;

means (6) for measuring the concentration of the ingredient in the mixed liquid and providing an output signal (61) corresponding to the measured concentration;

characterized by the apparatus further comprising:

means (7) for receiving said output signal, for varying the speed of said at least one speed-variable tube pump (2) to change the dilution ratio of the mixed liquid, for determining from the measured concentrations before and resulting from such change of dilution ratio and from the speed change of the tube pump the value of the dilution ratio before or resulting from the change of dilution ratio and determining from such value the concentration of the predetermined ingredient in the first liquid.

## Patentanspruch

Vorrichtung zum Überwachen der Konzentration eines bestimmten Bestandteiles in einer ersten Flüssigkeit (8) mit einer ersten Schlauchpumpe (2) zum Fördern eines der ersten Flüssigkeit zum Erhalt einer Flüssigkeitsmischung (10) zuzumischenden Verdünnungsmittels (9), mit einer zweiten Schlauchpumpe (3) zum Fördern der ersten Flüssigkeit oder Flüssigkeitsmischung, mit ersten und zweiten Mitteln (4, 5) zum Antreiben der ersten und der zweiten Schlauchpumpe, wobei die Antriebsgeschwindigkeit mindestens einer der Pumpen veränderbar ist, sowie mit einem Mittel (6) zur Bestimmung der Konzentration des bestimmten Bestandteiles in der Flüssigkeitsmischung, wobei das Mittel ein der gemessenen Konzentration entsprechendes Ausgangssignal (61) abgibt, dadurch gekennzeichnet, daß die Vorrichtung weiter versehen ist mit Mitteln (7) zur Aufnahme des Ausgangssignals, zum Verändern der Antriebsgeschwindigkeit zumindest der einen in ihrer Antriebsgeschwindigkeit veränderbaren Schlauchpumpe (2) zur Änderung des Verdünnungsverhältnisses der Flüssigkeitsmischung, sowie zum Bestimmen des Verdünnungsverhältnisses vor oder infolge der Änderung des Verdünnungsverhältnisses aus den gemessenen Konzentration vor und infolge einer solchen Änderung des Verdünnungsverhältnisses und aus der Veränderung der Antriebsgeschwindigkeit der Schlauchpumpe und Bestimmung der Konzentration des bestimmten Bestandteils in der ersten Flüssigkeit aus diesem Wert.

**Revendication**

Appareil pour contrôler la concentration d'un ingrédient prédéterminé dans un premier liquide (8), l'appareil comprenant:

une première pompe à tube (2) pour alimenter du diluant (9) à être mélangé au premier liquid pour obtenir un liquide mélangé (10);

une deuxième pompe à tube (3) pour alimenter le premier liquide ou le liquide mélangé;

des premier et second moyens (4, 5) pour enclencher respectivement la première ou la deuxième pompe à tube, la vitesse d'au moins une des pompes étant variable;

des moyens (6) pour mesurer la concentration de l'ingrédient dans le liquide mélangé et pour émettre un signal de sortie (61) correspondant à la concentration mésurée;

caractérisé en ce que l'appareil comprend en outre:

des moyens (7) pour recevoir ledit signal de sortie, pour varier la vitesse de ladite au moins une pompe à tube (2) à vitesse variable pour modifier le degré de dilution du liquide mélangé, pour déterminer à base des concentrations mesurées avant et résultant d'une telle modification du degré de dilution et par la variation de la vitesse de la pompe à tube la valeur du degré de dilution avant ou résultant de la modification du degré de dilution et déterminant d'une telle valeur la concentration de l'ingrédient prédéterminé dans le premier liquide.

FIG. 1.

FIG.2.

FIG.3.

(a)

(b)

FIG.4.